# EUROPÄISCHE PATENTSCHRIFT

(11) **EP 2 268 629 B1**
(45) Veröffentlichungstag und Bekanntmachung des Hinweises auf die Patenterteilung: **03.07.2013**
(21) Anmeldenummer: 09737886.3
(22) Anmeldetag: 29.04.2009
(51) Int. Cl.: C07D 311/92, G02B 5/23

(54) **PHOTOCHROME NAPHTHOPYRANE MIT DOPPELT VERBRÜCKTER TERPHENYL-UNTEREINHEIT**
PHOTOCHROMIC NAPHTHOPYRANES HAVING A DOUBLE-BRIDGED TERPHENYL SUB-UNIT
NAPHTOPYRANNES PHOTOCHROMIQUES AYANT UN SOUS-MOTIF TERPHÉNYLE DOUBLEMENT PONTÉ

(30) Priorität: 30.04.2008 DE 102008021823
(43) Veröffentlichungstag der Anmeldung: 05.01.2011
(73) Patentinhaber: Rodenstock GmbH, 80687 München (DE)
(72) Erfinder: MELZIG, Manfred, 82234 Wessling (DE); ROHLFING, Yven Dr., 81547 München (DE); WEIGAND, Udo Dr., 81247 München (DE)
(74) Vertreter: Hock, Joachim
(86) Internationale Anmeldenummer: PCT/EP2009/003113
(87) Internationale Veröffentlichungsnummer: WO 2009/132842

(56) Entgegenhaltungen:
- WO-A-2005/047277
- FR-A- 2 783 248

## Beschreibung

Die vorliegende Erfindung betrifft photochrome Naphthopyrane mit doppelt verbrückter Terphenyl-Untereinheit sowie deren Verwendung in Kunststoffen aller Art, insbesondere für ophthalmische Zwecke.

Seit langem sind verschiedene Farbstoffklassen bekannt, die bei Bestrahlung mit Licht bestimmter Wellenlängen, insbesondere Sonnenstrahlen, reversibel ihre Farbe wechseln. Dies geht darauf zurück, daß diese Farbstoffmoleküle durch Lichtenergie in einen angeregten Zustand übergehen, den sie bei Unterbrechung der Energiezufuhr wieder verlassen und in ihren Ausgangszustand zurückkehren. Zu diesen photochromen Farbstoffen gehören verschiedene Pyransysteme, die im Stand der Technik mit unterschiedlichen Grundsystemen und Substituenten bereits beschrieben wurden.

Pyrane, speziell Naphthopyrane und von diesen abgeleitete größere Ringsysteme, sind derzeit die am meisten bearbeitete Klasse photochromer Verbindungen. Obwohl bereits im Jahr 1966 erstmals zum Patent angemeldet (US 3,567,605), konnten erst in den 90er Jahren Verbindungen entwickelt werden, die für den Einsatz in Brillengläsern geeignet erschienen. Eine geeignete Verbindungsklasse von Pyranen sind zum Beispiel die 2,2-Diaryl-2H-naphtho[1,2-b]pyrane oder die 3,3-Diaryl-3H-naphtho[2,1-b]pyrane, die in angeregter Form verschiedene Färbungen, wie Gelb, Orange oder Rotorange, zeigen.

Als weitere Verbindungsklasse photochromer Verbindungen sind höher annellierte Pyrane von Interesse, die aufgrund ihres größeren Ringsystems längerwellig absorbieren und rote, violette und blaue Farbtöne ergeben. Diese können entweder von den 2H-Naphtho[1,2-b]pyranen oder den 3H-Naphtho[2,1-b]pyranen abgeleitete Systeme sein, die durch Annellierung an der f-Seite aus den jeweiligen Naphthopyran-Systemen hervorgehen.

Diarylchromene, insbesondere Naphthopyrane oder heterozyklisch annellierte Benzopyrane, die in 6-Stellung des Benzopyrans mit einem Phenylring oder allgemeiner einem aromatischen oder heteroaromatischen Ring substituiert sind, welcher zusätzlich über die 5-Stellung des Benzopyrans über mindestens ein Kohlenstoffatom, Sauerstoffatom oder Stickstoffatom verbrückt ist, sind derzeit die vielversprechendsten photochromen Verbindungen.

Wird diese Verbrückung nur über ein Atom erzeugt, so ergibt sich ein an das Benzopyran annellierter Fünfring. Beispiele finden sich für ein Kohlenstoffatom in US 5,645,767,- US 5,723,072 sowie US 5,955,520 und für ein Sauerstoffatom in US 6,018,059.

In US 5,723,072 kann zusätzlich an diesem Grundsystem ein un-, mono- oder disubstituierter heterozyklischer Ring an der g-, h-, i-, n-, o- oder p-Seite des Indenonaphthopyrans annelliert sein. Es werden demzufolge Indeno[1,2-f]naphtho[1,2-b]pyrane mit einer sehr großen Variationsbreite an möglichen Substituenten offenbart.

In WO 96/14596, WO 99/15518, US 5,645,767, WO 98/32037 und US 5,698,141 werden vom 2H-Naphtho[1,2-b]pyran abgeleitete photochrome indenoannellierte Naphthopyran-Farbstoffe, die sie enthaltenden Zusammensetzungen sowie ein Verfahren zu ihrer Herstellung offenbart. In US 5,698,141 kann zusätzlich an diesem Grundsystem ein un-, mono- oder disubstituierter heterozyklischer Ring an der g-, h-, i-, n-, o- oder p-Seite des Indenonaphthopyrans annelliert sein. Von der jeweils sehr umfangreichen Substituentenliste sind auch ganz spezielle Spiro-Verbindungen umfaßt, und zwar solche Systeme mit einer spiroheterozyklischen Gruppe, worin einschließlich des Spiroatoms an der 13-Position des Grundsystems ein 5- bis 8-gliedriger Ring, der stets zwei Sauerstoffatome enthält, vorhanden ist. Eine weitere Ausführungsform des Spiroringes findet sich in der japanischen Anmeldung 344762/2000.

Wird diese Verbindung über zwei Atome erzeugt, so ergibt sich ein annellierter Sechsring mit allein für C, O und N vielfältigen Möglichkeiten. Verbindungen mit C=O und N-R (Lactam-Brücke) werden in US 6,379,591 beschrieben. Verbindungen mit einer unsubstituierten CH₂-CH₂-Brücke sowie einem annellierten Heterocyclus in 7,8-Stellung des zugrundeliegenden Benzopyrans sind in US 6,426,023 offenbart.

US 6,506,538 beschreibt die carbocyclischen Analogverbindungen, bei denen die H-Atome in der Brücke durch OH, (C₁ - C₆) Alkoxy oder zwei H-Atome an einem C-Atom durch =O ersetzt sein können. Alternativ kann auch eines der Kohlenstoffatome in der zweigliedrigen Brücke durch Sauerstoff ersetzt sein. Diese Verbindungen sind neben weiteren in WO 00/02884 beschrieben.

Wird diese Verbindung durch drei Atome erzeugt, ergibt sich ein annellierter 7-Ring mit sehr vielen Variationsmöglichkeiten durch Einfügen von Heteroatomen. Verbindungen mit einer CH₂-CH₂-CH₂-Brücke sind in US 6,558,583 beschrieben. Auch hier können die H-Atome in der Brücke durch OH, (C₁-C₆)-Alkyl oder (C₁ - C₆)-Alkoxy oder zwei Wasserstoffatome an einem C-Atom durch =O ersetzt sein. Sie absorbieren bei gleichem Substitutionsmuster kürzerwellig als die annellierten 6-Ringe.

US 2004/0094753 beschreibt sowohl Verbindungen mit 2- wie mit 3-atomiger Brücke. Die zweiatomige (Kohlenstoff-)Brücke ist dabei zusätzlich mit einem Carbon- bzw. Heterocyclus annelliert. Die dreiatomige Brücke enthält drei C-Atome oder zwei C-Atome und ein O-Atom ohne zusätzliche Annellierung. Beide Ringe können vielfältige Substituenten tragen.

Die verschiedenen, im Stand der Technik verfügbaren photochromen Farbstoffe haben jedoch Nachteile, die bei der Verwendung in Sonnenschutzgläsern den Tragekomfort des Brillenträgers wesentlich beeinträchtigen. Zum einen weisen die Farbstoffe eine nicht ausreichend langwellige Absorption im angeregten wie im nicht angeregten Zustand auf. Zum anderen liegt häufig eine zu hohe Temperaturempfindlichkeit der Eindunkelung vor, wobei gleichzeitig eine zu langsame Aufhellung eintreten kann. Darüber hinaus besitzen die im Stand der Technik verfügbaren Farbstoffe oft eine ungenügende Lebensdauer und erlauben damit nur eine geringe Haltbarkeit der Sonnenschutzgläser. Letzteres macht sich in schnell nachlassender Leistung und/oder starker Vergilbung bemerkbar.

Der vorliegenden Erfindung liegt daher die Aufgabe zugrunde, eine Klasse photochromer Verbindung bereitzustellen, die deutlich verbesserte Eigenschaften gegenüber den im Stand der Technik beschriebenen Strukturen besitzen sollen. Diese sind in der Kombination von langwelligem Absorptionsmaximum der geschlossenen Form mit steiler Kante zum sichtbaren Wellenlängenbereich, hoher Eindunkelungsleistung, sehr schneller Aufhellreaktion und sehr guter Lichtbeständigkeit zu finden.

Diese Aufgabe wird durch die in den Ansprüchen gekennzeichneten Gegenstände gelöst.

Insbesondere werden photochrome Naphthopyrane mit doppelt verbrückter Terphenyl-Untereinheit mit der allgemeinen Formel (I) bereitgestellt: 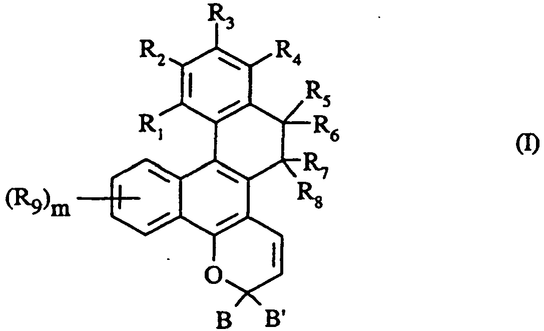 worin
mindestens einer der Reste R₂, R₃ oder R₄, vorzugsweise R₂ oder R₃, die folgende Einheit (A) darstellt, 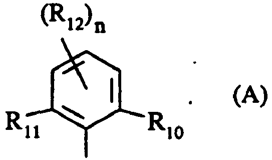 mit der Maßgabe, daß R₁₀ oder R₁₁ zusammen mit einem zur Kopplungsstelle ortho-ständigen Rest R₁, R₂, R₃ oder R₄ eine Verbrückung bildet, oder R₁₀ und R₁₁ im Falle einer Kopplung der vorstehenden Einheit (A) über R₂ jeweils zusammen mit beiden zur Kopplungsstelle ortho-ständigen Resten R₁ und R₃ bzw. im Falle einer Kopplung der vorstehenden Einheit (A) über R₃ mit beiden zur Kopplungsstelle ortho-ständigen Resten R₂ und R₄ zwei Verbrückungen bilden, wobei die Verbrückung über die Reste R₁₀ bzw. R₁₁ jeweils eine solche ist, ausgewählt aus der Gruppe, bestehend aus -CR₁₃R₁₄-, -O-, -S-, -N(Ph)-, -N(C₁-C₆ Alkyl)-, -O-CR₁₃R₁₄-, -S-CR₁₃R₁₄-, -CR₁₃R₁₄-CR₁₃R₁₄-, -CR₁₅=CR₁₆- oder -CR₁₅=N-,
und worin
die Reste R₁, R₂, R₃, R₄, R₁₀ und R₁₁, sofern sie nicht eine Verbrückung bilden, sowie die Reste R₉ und R₁₂ jeweils unabhängig voneinander einen Substituenten darstellen, ausgewählt aus der Gruppe α, bestehend aus einem Wasserstoffatom, einem (C₁ - C₆)-Alkylrest, einem (C₁-C₆)-Thioalkylrest, einem (C₃-C₇)-Cycloalkylrest, der ein oder mehrere Heteroatome, wie beispielsweise O oder S, aufweisen kann, einem (C₁ - C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können; oder
die Reste R₁ und R₂ im Falle einer Kopplung der vorstehenden Einheit (A) über R₄ einen annellierten, unsubstituierten, mono- oder disubstituierten Benzo-, Naphtho- oder Pyridoring darstellen, dessen Substituenten aus der Gruppe α ausgewählt sein können;
oder zwei zueinander orthoständige Reste R₉ bzw. zwei zueinander orthoständige Reste R₁₂ eine an den aromatischen Ring gebundene -D-(CH₂)ₖ-E- Gruppe oder -D-(C(CH₃)₂)ₖ-E- Gruppe mit k = 1 oder 2 bilden, wobei D und E unabhängig voneinander aus Sauerstoff, Schwefel, CH₂, C(CH₃)₂ oder C(C₆H₅)₂ ausgewählt sind, und wobei an diese -D-(CH₂)ₖ-E- Gruppe wiederum ein Benzoring annelliert sein kann;
oder zwei zueinander orthoständige Reste R₉ bzw. zwei zueinander orthoständige Reste R₁₂ einen unsubstituierten, mono- oder disubstituierten Benzo- oder Pyridoring darstellen, dessen Substituenten aus der Gruppe α ausgewählt sein können;
die Reste R₅, R₆, R₇ und R₈ jeweils unabhängig voneinander aus der Gruppe α ausgewählt sind, oder
die Reste R₅ und R₆ bzw. R₇ und R₈ zusammen unter Einbeziehung des Spiro-Kohlenstoffatoms einen 3- bis 8-gliedrigen carbo- oder heteromonozyklischen Spiro-Ring, der gegebenenfalls einen oder mehrere Substituenten aus der Gruppe α trägt, bilden, an den ein bis drei aromatische oder heteroaromatische Ringsysteme annelliert sein können, wobei das Ringsystem unabhängig voneinander aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist, wobei zwei benachbarte annellierte Ringsysteme auch durch eine ortho,ortho'-Brücke miteinander verknüpft sein können,oder
die Reste R₅ und R₆ bzw. R₇ und R₈ zusammen unter Einbeziehung des Spiro-Kohlenstoffatoms einen 7- bis 12-gliedrigen carbo-bizyklischen Spiro-Ring oder einen 7- bis 12-gliedrigen carbo-trizyklischen Spiro-Ring bilden, die jeweils gegebenenfalls ein oder mehrere Substituenten aus der Gruppe α tragen können,
oder
die Reste R₅ und R₆ zusammen mit R₄ einen annellierten, unsubstituierten, mono- oder disubstituierten Benzo-, Naphtho- oder Pyridoring darstellen, dessen Substituenten aus der Gruppe α ausgewählt sein können,
die Reste R₁₃, R₁₄, R₁₅ und R₁₆ jeweils unabhängig voneinander einen Substituenten darstellen, ausgewählt aus der Gruppe α, oder
die Reste R₁₃ und R₁₄ zusammen mit einem zur Kopplungsstelle meta-ständigen Rest R₁₂ einen annellierten, unsubstituierten, mono- oder disubstituierten Benzo-, Naphtho- oder Pyridoring darstellen, dessen Substituenten aus der Gruppe α ausgewählt sein können, wobei dies bei einer Verbrückung mittels der Gruppen -O-CR₁₃R₁₄- bzw. -S-CR₁₃R₁₄- nur möglich ist, wenn CR₁₃R₁₄ direkt an den Phenylring, der R₁₂ trägt, gebunden ist, oder
im Falle einer Kopplung der vorstehenden Einheit (A) über R₃ und einer Verbrückung über R₂ die Reste R₁₃ und R₁₄ zusammen mit R₁ bzw. im Falle einer Kopplung der vorstehenden Einheit (A) über R₄ und einer Verbrückung über R₃ die Reste R₁₃ und R₁₄ zusammen mit R₂ bzw. im Falle einer Kopplung der vorstehenden Einheit (A) über R₂ und einer Verbrückung über R₃ die Reste R₁₃ und R₁₄ zusammen mit R₄ einen annellierten, unsubstituierten, mono- oder disubstituierten Benzo-, Naphtho- oder Pyridoring darstellen, dessen Substituenten aus der Gruppe α ausgewählt sein können, oder
im Falle einer Kopplung der vorstehenden Einheit (A) über R₃ und einer Verbrückung über R₄ die Reste R₁₃ und R₁₄ zusammen mit R₅ und R₆ einen annellierten, unsubstituierten, mono- oder disubstituierten Benzo-, Naphtho- oder Pyridoring darstellen, dessen Substituenten aus der Gruppe α ausgewählt sein können, wobei dies bei einer Verbrückung mittels der Gruppen -O-CR₁₃R₁₄- bzw. -S-CR₁₃R₁₄- nur möglich ist, wenn CR₁₃R₁₄ direkt an den Phenylring, der auch CR₅R₆ trägt, gebunden ist, oder
die Reste R₁₅ und R₁₆ einen annellierten, unsubstituierten, mono- oder disubstituierten Benzo-, Naphtho- oder Pyridoring darstellen, dessen Substituenten aus der Gruppe α ausgewählt sein können;
m 0, 1, 2, 3 oder 4 ist und n 0, 1, 2 oder 3 ist;
B und B' unabhängig voneinander aus einer der folgenden Gruppen a), b) oder c) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist;
   wobei die Substituenten der Aryl- oder Heteroarylreste in a) und b) solche sind, ausgewählt aus der vorstehend definierten Gruppe α oder der Gruppe χ, bestehend aus Hydroxy, am Phenylring un-, mono- oder disubstituiertem 2-Phenylethenyl, am Phenylring un-, mono- oder disubstituiertem (Phenylimino)methylen, am Phenylring un-, mono- oder disubstituiertem (Phenylmethylen)imino, Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können; oder wobei zwei direkt benachbarte Substituenten eine Y-(CX₂)ₚ-Z-Gruppierung darstellen, wobei p = 1, 2 oder 3 ist, X Wasserstoff, CH₃ oder C₆H₅ sein kann und Y und Z unabhängig voneinander Sauerstoff, Schwefel, N-(C₁-C₆)-Alkyl, N-C₆H₅, CH₂, C(CH₃)₂ oder C(C₆H₅)₂ sein können, wobei zwei oder mehrere benachbarte Kohlenstoffatome dieser Y-(CX₂)ₚ-Z-Gruppierung jeweils unabhängig voneinander auch Teil eines daran annellierten Benzo-Ringsystems sein können, welches jeweils wiederum einen oder mehrere Substituenten, ausgewählt aus der Gruppe α oder der Gruppe χ, aufweisen kann;
   oder
c) B und B' zusammen mit dem benachbarten Kohlenstoffatom des Pyran-Ringes einen un-, mono- oder disubstituierten 9,10-Dihydroanthracen-, Fluoren-, Thioxanthen-, Xanthen-, Benzo[b]fluoren-, 5H-Dibenzo[a,d]cyclohepten oder Dibenzosuberon-Rest oder einen gesättigten Kohlenwasserstoffrest, der (C₃-C₁₂)-spiro-monozyklisch, (C₇-C₁₂)-spiro-bizyklisch bzw. (C₇-C₁₂)-spiro-trizyklisch ist, bilden, wobei die Substituenten der ungesättigten Cyclen unabhängig voneinander aus der Gruppe α oder der Gruppe χ ausgewählt sein können.

Die erfindungsgemäßen photochromen Naphthopyrane mit doppelt verbrückter Terphenyl-Untereinheit weisen im Vergleich zu derzeit im Stand der Technik verfügbaren Systemen ein verbessertes Eigenschaftsprofil, insbesondere eine verbesserte Kombination von sehr guter Lebensdauer sowie schneller Aufhellungsgeschwindigkeit auf. Die erfindungsgemäßen Verbindungen zeigen eine ausgewogene Balance von langwelligem Absorptionsmaximum, hoher Eindunkelungsleistung, sehr schneller Aufhellreaktion und sehr guter Lichtbeständigkeit.

Die Reste R₅ und R₆ bzw. R₇ und R₈ können zusammen unter Einbeziehung des Spiro-Kohlenstoffatoms einen 3- bis 8-gliedrigen carbo- oder heterozyklischen Ring bilden, an den ein bis drei aromatische oder heteroaromatische Ringsysteme annelliert sein können, wobei das Ringsystem aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist. Dabei können auch zwei benachbarte annellierte Ringsysteme durch eine ortho,ortho'-Brücke, vorzugsweise eine Ethylen- oder eine 1,2-Ethendiyl-Brücke miteinander verknüpft sein, so daß beispielsweise im letzteren Fall folgende Struktureinheit vorliegt:

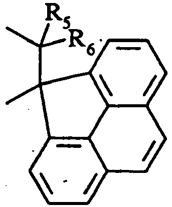

In einer bevorzugten Ausführungsform sind jedoch die Reste Reste R₅ und R₆ bzw. R₇ und R₈ jeweils unabhängig voneinander aus der Gruppe α ausgewählt.

Wenn B bzw. B' für einen gesättigten Kohlenwasserstoffrest steht, der C₃-C₁₂ spiro-monozyklisch, C₇-C₁₂ spiro-bizyklisch oder C₇-C₁₂ spiro-trizyklisch ist, so werden unter C₃-C₁₂ spiro-monozyklisch ein dem Fachmann geläufiger 3-gliedriger bis 12-gliedriger Ring verstanden. Auch C₇-C₁₂ spiro-bizyklische Systeme sind einem Fachmann wohlbekannt. Beispielhaft kann hier wiederum Norbornan, Norbornen, 2,5-Norbornadien, Norcaran und Pinan genannt werden. Ein beispielhaftes C₇-C₁₂ spiro-trizyklisches System ist Adamantan.

In einer weiteren bevorzugten Ausführungsform sind die Reste B und B' unabhängig voneinander aus der Gruppe a), wie vorstehend definiert, ausgewählt.

Die Substituenten der Gruppe χ, welche Stickstoffatome aufweisen bzw. Amingruppen tragen, sind über diese an den Phenyl, Naphthyl- bzw. Phenanthrylrest der Gruppe a) gebunden.

Wenn bezüglich der Substituenten der Gruppe χ, welche an den Phenyl, Naphthyl- bzw. Phenanthrylrest der Gruppe a) für die Reste B bzw. B' gebunden sein können, zwei oder mehrere benachbarte Kohlenstoffatome dieser Y-(CX₂)ₚ-Z-Gruppierung jeweils unabhängig voneinander Teil eines daran annellierten Benzo-Ringsystems sein können, so bedeutet dies, dass dann die beiden Methylenkohlenstoffatome (- CH₂- CH₂-) Teil eines annellierten Ringsystems werden. Wenn beispielsweise zwei oder drei Benzoringe annelliert sind, so können beispielsweise hier dann folgende Struktureinheiten vorliegen, wie nachstehend angeführt.

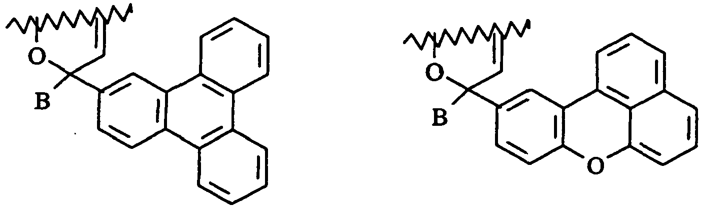

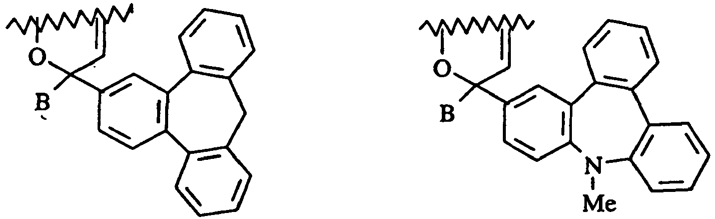

Selbstverständlich kann aber auch nur ein, über zwei benachbarte Kohlenstoffatome dieser Y-(CX₂)ₚ-Z-Gruppierung annellierter Benzoring vorliegen.

Bevorzugte photochrome Naphthopyrane mit doppelt verbrückter Terphenyl-Untereinheit gemäß der vorliegenden Erfindung weisen die nachfolgenden allgemeinen Formeln (II) bzw. (III) auf: 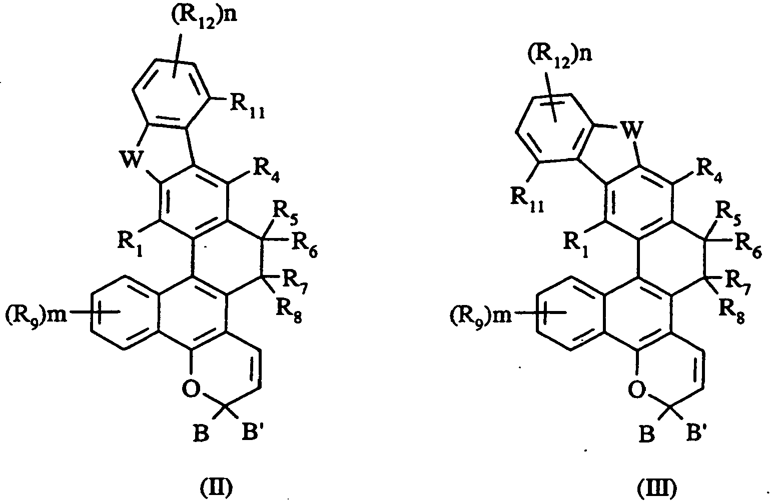 worin m, n, B, B', R₁ sowie R₄ bis R₁₂ wie vorgenannt definiert sind und W die vorstehend angeführte Verbrückung symbolisiert (die Brücke wird in diesen beiden Fällen von den Resten R₂ und R₁₀ gebildet und ist wie vorgenannt definiert, d.h. ausgewählt aus der Gruppe, bestehend aus bestehend aus -CR₁₃R₁₄-, -O-, -S-, -N(Ph)-, -N(C₁-C₆ Alkyl)-, -O-CR₁₃R₁₄-, -S-CR₁₃R₁₄-, -CR₁₃R₁₄-CR₁₃R₁₄-, -CR₁₅=CR₁₆- oder -CR₁₅=N-).

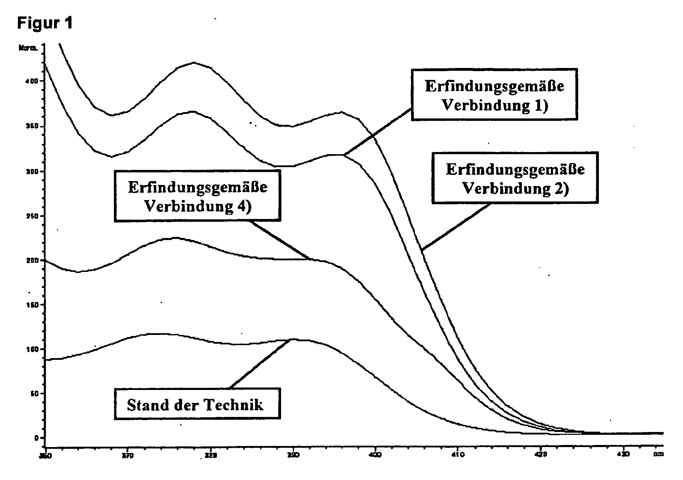

Im Vergleich zum Stand der Technik, d.h. US 6,506,538, weisen die erfindungsgemäßen Verbindungen - bei sonst gleichen Substituenten B, B', R₁ sowie R₄ bis R₉ - eine längerwellige und vor allem hyperchrome (intensivere) Absorptionsbande sowohl im nicht angeregten als auch im angeregten Zustand auf. Eine längerwellige und intensivere Absorption im nicht angeregten Zustand hat beim Einbringen der photochromen Farbstoffe z.B. in Kunststoff-Brillengläsern zwei wichtige Vorteile. Zum einen reagieren die erfindungsgemäßen Verbindungen auch dann, wenn bei ungünstigen atmosphärischen Bedingungen nur sehr langwelliges UV-Sonnenlicht (ab 380 nm) einfällt. Aus Figur 1 ist ersichtlich, dass die erfindungsgemäßen Verbindungen in der nicht angeregten Form bei Wellenlängen von größer als 370 nm deutlich intensiver absorbieren als im Vergleich zu Verbindungen des Standes der Technik. Dadurch zeigen die erfindungsgemäßen photochromen Verbindungen auch bei ungünstigen Bedingungen eine sehr gute Eindunklungsleistung. Zum anderen wird dadurch automatisch ein vollständiger UV-Schutz bis 400 nm erreicht, da die erfindungsgemäßen Verbindungen das einfallende UV-Licht komplett absorbieren. Ein Zusatz von UV-Absorbern bei der Herstellung von Sonnenschutzgläsern erübrigt sich. Dies ist ein großer Vorteil, da zugemischte UV-Absorber immer auch einen Teil des Anregungslichts absorbieren, so daß Gläser mit UV-Absorber immer weniger stark eindunkeln als ohne.

Die Struktur der in der Figur 1 dargestellten erfindungsgemäßen Verbindungen sowie deren längstwellige Absorptionsmaxima in der angeregten Form sind aus der folgenden Tabelle 1 ersichtlich (im Vergleich zum Stand der Technik aus US 6,506,538):

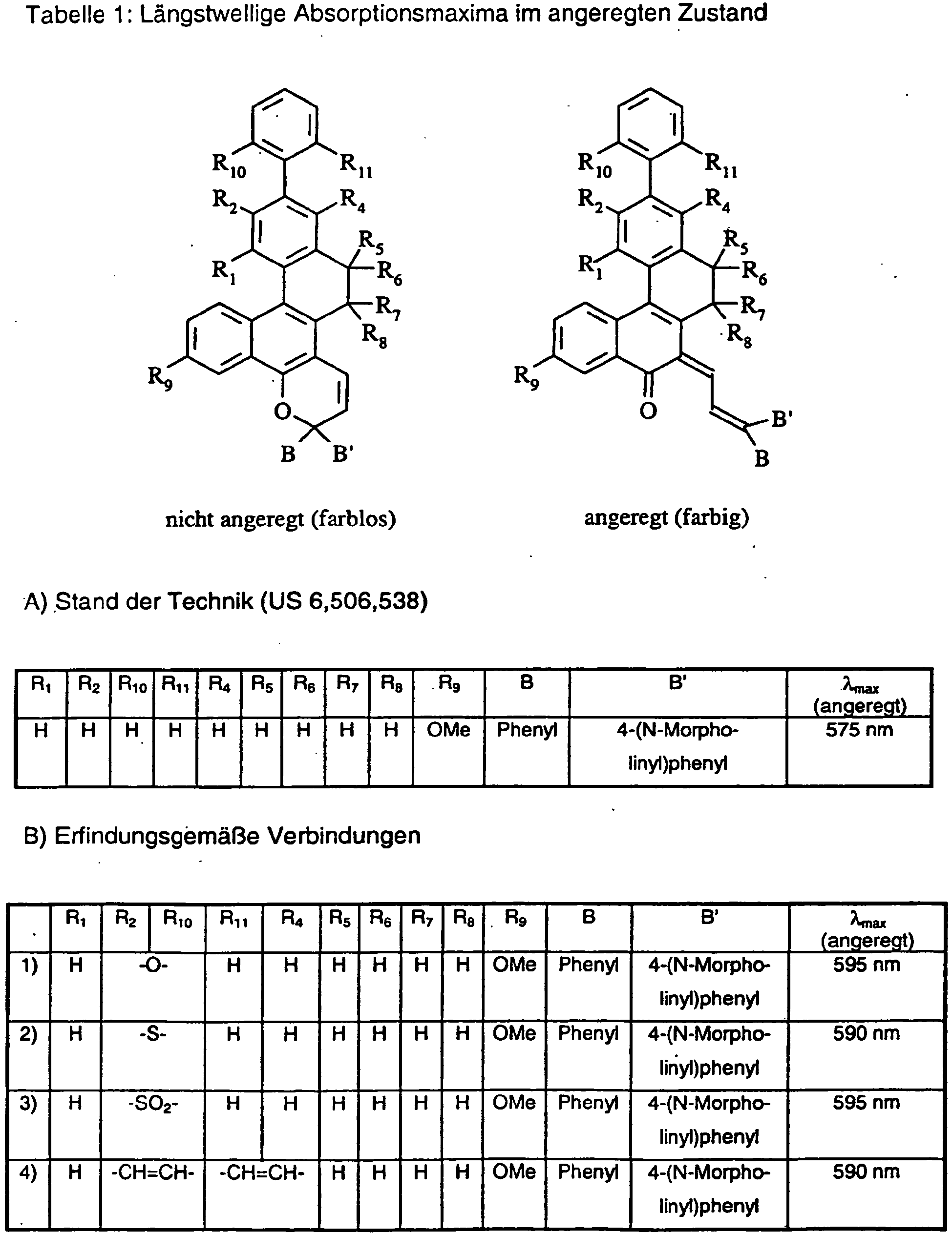

Zur Messung der Eigenschaften der erfindungsgemäßen photochromen Farbstoffe sowie der Verbindung aus dem Stand der Technik (s.o.) wurden jeweils 500 ppm des Farbstoffes in einer Acrylatmonomer-Matrix gelöst und nach Zusatz eines Polymerisations-Initiators mit Hilfe eines Temperaturprogrammes thermisch polymerisiert. Die Transmisionseigenschaften der so hergestellten Kunststoffgläser (Dicke 2mm) wurden anschließend nach DIN EN ISO 8980-3 vermessen.

Die erfindungsgemäßen Verbindungen können in Kunststoffmaterialien bzw. Kunststoffgegenständen jeglicher Art und Form für eine Vielzahl von Einsatzzwecken, für die photochromes Verhalten von Bedeutung ist, verwendet werden. Dabei können ein Farbstoff gemäß der vorliegenden Erfindung oder ein Gemisch solcher Farbstoffe eingesetzt werden. Beispielsweise können die erfindungsgemäßen photochromen Naphthopyrane mit doppelt verbrückter Terphenyl-Untereinheit in Linsen, insbesondere ophthalmischen Linsen, Gläsern für Brillen aller Art, wie beispielsweise Skibrillen, Sonnenbrillen, Motorradbrillen, Visieren von Schutzhelmen, und dergleichen eingesetzt werden. Ferner können die erfindungsgemäßen photochromen Naphthopyrane mit doppelt verbrückter Terphenyl-Untereinheit beispielsweise auch als Sonnenschutz in Fahrzeugen und Wohnräumen in Form von Fenstern, Schutzblenden, Abdeckungen, Dächern oder dergleichen verwendet werden.

Zur Herstellung von solchen photochromen Gegenständen können die erfindungsgemäßen photochromen Naphthopyrane mit doppelt verbrückter Terphenyl-Untereinheit durch verschiedene, im Stand der Technik beschriebene Verfahren, wie bereits in WO 99/15518 angegeben, auf ein Polymermaterial, wie ein organisches Kunststoffmaterial, aufgebracht oder darin eingebettet werden.

Es werden dabei sogenannte Massefärbungs- und Oberflächenfärbungsverfahren unterschieden. Ein Massefärbungsverfahren umfasst beispielsweise das Auflösen oder Dispergieren der photochromen Verbindung oder Verbindungen gemäß der vorliegenden Erfindung in einem Kunststoffmaterial, z.B. durch die Zugabe der photochromen Verbindung(en) zu einem monomeren Material, bevor die Polymerisation erfolgt. Eine weitere Möglichkeit zur Herstellung eines photochromen Gegenstands ist die Durchdringung des oder der Kunststoffmaterialien mit der (den) photochromen Verbindung(en) durch Eintauchen des Kunststoffmaterials in eine heiße Lösung des oder der photochromen Farbstoffe gemäß der vorliegenden Erfindung oder beispielsweise auch ein Thermotransferverfahren. Die photochrome(n) Verbindung(en) kann bzw. können beispielsweise auch in Form einer separaten Schicht zwischen aneinandergrenzenden Schichten des Kunststoffmaterials, z.B. als Teil eines polymeren Films, vorgesehen werden. Ferner ist auch ein Aufbringen der photochromen Verbindung(en) als Teil einer auf der Oberfläche des Kunststoffmaterials befindlichen Beschichtung möglich. Der Ausdruck "Durchdringung" soll dabei die Migration der photochromen Verbindung(en) in das Kunststoffmaterial, z.B. durch den lösungsmittelunterstützten Transfer der photochromen Verbindung(en) in eine Polymermatrix, Dampfphasentransfer oder andere derartige Oberflächendiffusionsvorgänge, bedeuten. Vorteilhafterweise können solche photochromen Gegenstände, wie z.B. Brillengläser, nicht nur mittels der üblichen Massefärbung, sondern in gleicher Weise auch mittels Oberflächenfärbung hergestellt werden, wobei bei der letzteren Variante eine überraschend geringere Migrationsneigung erzielt werden kann. Dies ist vor allem bei nachfolgenden Veredelungsschritten von Vorteil, da - z.B. bei einer Antireflexbeschichtung durch die geringere Rückdiffusion im Vakuum - Schichtablösungen und ähnliche Defekte drastisch verringert werden.

Insgesamt können auf Basis der erfindungsgemäßen photochromen Naphthopyrane mit doppelt verbrückter Terphenyl-Untereinheit beliebig kompatible (in chemischer Hinsicht und farblicher Art und Weise verträgliche) Färbungen, d.h. Farbstoffe, auf das Kunststoffmaterial aufgebracht oder in es eingebettet werden, um sowohl ästhetischen Gesichtspunkten als auch medizinischen oder modischen Aspekten zu genügen. Der oder die spezifisch ausgewählte(n) Farbstoff(e) kann bzw. können demzufolge, abhängig von den beabsichtigten Wirkungen sowie Anforderungen, variieren.

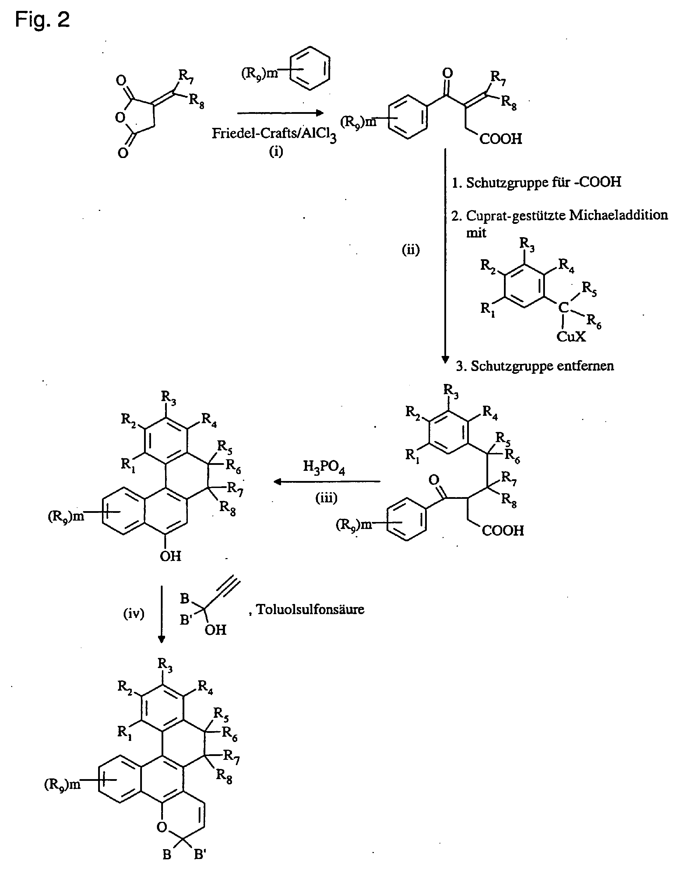

Die erfindungsgemäßen photochromen Verbindungen lassen sich gemäß folgendem beispielhaften Syntheseschema, wie in Figur 2 aufgezeigt, herstellen.

Geeignet substituierte Methylidensuccinanhydride werden in einem ersten Schritt einer Friedel-Crafts-Reaktion mit geeignet substituierten Benzolderivaten (Schritt (i)) unterworfen. Die -COOH Gruppe des daraus resultierenden Intermediats wird anschließend geschützt und dieses Intermediat einer Cuprat-gestützten Michael-Addition mit entsprechend substituierten Benzylderivaten unterworfen (Schritt (ii)). Nach Entfernung der Carbonsäure-Schutzgruppe werden via intramolekularer Cyclisierung mittels Phosphorsäure entsprechend substituierte 9,10-Dihydrophenanthren-Derivate gebildet (Schritt (iii)). Anschließend werden diese substituierten Dihydrophenanthren-Derivate mit geeignet substituierten 2-Propin-1-ol-Derivaten gemäß Schritt (iv) zu den erfindungsgemäßen Verbindungen umgesetzt.

## Patentansprüche

1. Photochrome Naphthopyrane mit doppelt verbrückter Terphenyl-Untereinheit mit der allgemeinen Formel (I): 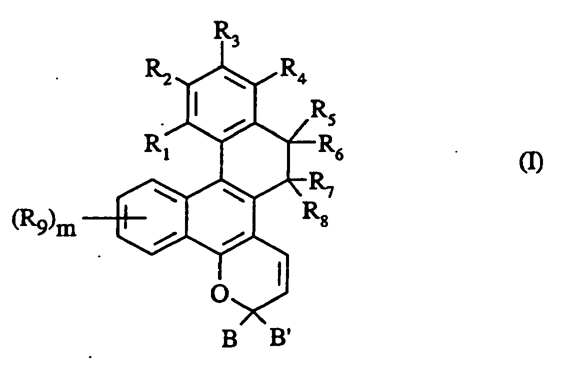 worin
mindestens einer der Reste R₂, R₃ oder R₄, vorzugsweise R₂ oder R₃, die folgende Einheit (A) darstellt, 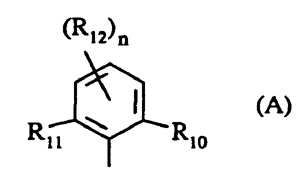 mit der Maßgabe, daß R₁₀ oder R₁₁ zusammen mit einem zur Kopplungsstelle ortho-ständigen Rest R₁, R₂, R₃ oder R₄ eine Verbrückung bildet, oder R₁₀ und R₁₁ im Falle einer Kopplung der vorstehenden Einheit (A) über R₂ jeweils zusammen mit beiden zur Kopplungsstelle ortho-ständigen Resten R₁ und R₃ bzw. im Falle einer Kopplung der vorstehenden Einheit (A) über R₃ mit beiden zur Kopplungsstelle ortho-ständigen Resten R₂ und R₄ zwei Verbrückungen bilden, wobei die Verbrückung über die Reste R₁₀ bzw. R₁₁ jeweils eine solche ist, ausgewählt aus der Gruppe, bestehend aus -CR₁₃R₁₄-, -O-, -S-, -N(Ph)-, -N(C₁-C₆ Alkyl)-, -O-CR₁₃R₁₄-, -S-CR₁₃R₁₄-; -CR₁₃R₁₄-CR₁₃R₁₄-, -CR₁₅=CR₁₆- oder -CR₁₅=N-,
und worin
die Reste R₁, R₂, R₃, R₄, R₁₀ und R₁₁, sofern sie nicht eine Verbrückung bilden, sowie die Reste R₉ und R₁₂ jeweils unabhängig voneinander einen Substituenten darstellen, ausgewählt aus der Gruppe α, bestehend aus einem Wasserstoffatom, einem (C₁- C₆)-Alkylrest, einem (C₁-C₆)-Thioalkylrest, einem (C₃ - C₇)-Cycloalkylrest, der ein oder mehrere Heteroatome, wie beispielsweise O oder S, aufweisen kann, einem (C₁- C₆)-Alkoxyrest, einer Hydroxygruppe, einer Trifluormethylgruppe, Brom, Chlor, Fluor, einem un-, mono- oder disubstituiertem Phenyl-, Phenoxy-, Benzyl-, Benzyloxy-, Naphthyl- oder Naphthoxyrest, wobei die Substituenten wiederum aus der Gruppe α ausgewählt sein können; oder
die Reste R₁ und R₂ im Falle einer Kopplung der vorstehenden Einheit (A) über R₄ einen annellierten, unsubstituierten, mono- oder disubstituierten Benzo-, Naphtho- oder Pyridoring darstellen, dessen Substituenten aus der Gruppe α ausgewählt sein können;
oder zwei zueinander orthoständige Reste R₉ bzw. zwei zueinander orthoständige Reste R₁₂ eine an den aromatischen Ring gebundene -D-(CH₂)ₖ-E- Gruppe oder -D-(C(CH₃)₂)ₖ-E- Gruppe mit k = 1 oder 2 bilden, wobei D und E unabhängig voneinander aus Sauerstoff, Schwefel, CH₂, C(CH₃)₂ oder C(C₆H₅)₂ ausgewählt sind, und wobei an diese -D-(CH₂)ₖ-E- Gruppe wiederum ein Benzoring annelliert sein kann;
oder zwei zueinander orthoständige Reste R₉ bzw. zwei zueinander orthoständige Reste R₁₂ einen unsubstituierten, mono- oder disubstituierten Benzo- oder Pyridoring darstellen, dessen Substituenten aus der Gruppe α ausgewählt sein können;
die Reste R₅, R₆, R₇ und R₈ jeweils unabhängig voneinander aus der Gruppe α ausgewählt sind, oder
die Reste R₅ und R₆ bzw. R₇ und R₈ zusammen unter Einbeziehung des Spiro-Kohlenstoffatoms einen 3- bis 8-gliedrigen carbo- oder heteromonozyklischen Spiro-Ring, der gegebenenfalls einen oder mehrere Substituenten aus der Gruppe α trägt, bilden, an den ein bis drei aromatische oder heteroaromatische Ringsysteme annelliert sein können, wobei das Ringsystem unabhängig voneinander aus der Gruppe β, bestehend aus Benzol, Naphthalin, Phenanthren, Pyridin, Chinolin, Furan, Thiophen, Pyrrol, Benzofuran, Benzothiophen, Indol und Carbazol, die wiederum mit einem oder mehreren Substituenten aus der Grupppe α substituiert sein können, ausgewählt ist, wobei zwei benachbarte annellierte Ringsysteme auch durch eine ortho,ortho'-Brücke miteinander verknüpft sein können, oder
die Reste R₅ und R₆ bzw. R₇ und R₈ zusammen unter Einbeziehung des Spiro-Kohlenstoffatoms einen 7- bis 12-gliedrigen carbo-bizyklischen Spiro-Ring oder einen 7- bis 12-gliedrigen carbo-trizyklischen Spiro-Ring bilden, die jeweils gegebenenfalls ein oder mehrere Substituenten aus der Gruppe α tragen können,
oder
die Reste R₅ und R₆ zusammen mit R₄ einen annellierten, unsubstituierten, mono- oder disubstituierten Benzo-, Naphtho- oder Pyridoring darstellen, dessen Substituenten aus der Gruppe α ausgewählt sein können,
die Reste R₁₃, R₁₄, R₁₅ und R₁₆ jeweils unabhängig voneinander einen Substituenten darstellen, ausgewählt aus der Gruppe α, oder
die Reste R₁₃ und R₁₄ zusammen mit einem zur Kopplungsstelle meta-ständigen Rest R₁₂ einen annellierten, unsubstituierten, mono- oder disubstituierten Benzo-, Naphtho- oder Pyridoring darstellen, dessen Substituenten aus der Gruppe α ausgewählt sein können, wobei dies bei einer Verbrückung mittels der Gruppen -O-CR₁₃R₁₄- bzw. -S-CR₁₃R₁₄- nur möglich ist, wenn CR₁₃R₁₄ direkt an den Phenylring, der R₁₂ trägt, gebunden ist, oder
im Falle einer Kopplung der vorstehenden Einheit (A) über R₃ und einer Verbrückung über R₂ die Reste R₁₃ und R₁₄ zusammen mit R₁ bzw. im Falle einer Kopplung der vorstehenden Einheit (A) über R₄ und einer Verbrückung über R₃ die Reste R₁₃ und R₁₄ zusammen mit R₂ bzw. im Falle einer Kopplung der vorstehenden Einheit (A) über R₂ und einer Verbrückung über R₃ die Reste R₁₃ und R₁₄ zusammen mit R₄ einen annellierten, unsubstituierten, mono- oder disubstituierten Benzo-, Naphtho- oder Pyridoring darstellen, dessen Substituenten aus der Gruppe α ausgewählt sein können, oder
im Falle einer Kopplung der vorstehenden Einheit (A) über R₃ und einer Verbrückung über R₄ die Reste R₁₃ und R₁₄ zusammen mit R₅ und R₆ einen annellierten, unsubstituierten, mono- oder disubstituierten Benzo-, Naphtho- oder Pyridoring darstellen, dessen Substituenten aus der Gruppe α ausgewählt sein können, wobei dies bei einer Verbrückung mittels der Gruppen -O-CR₁₃R₁₄- bzw. -S-CR₁₃R₁₄- nur möglich ist, wenn CR₁₃R₁₄ direkt an den Phenylring, der auch CR₅R₆ trägt, gebunden ist, oder die Reste R₁₅ und R₁₆ einen annellierten, unsubstituierten, mono- oder disubstituierten Benzo-, Naphtho- oder Pyridoring darstellen, dessen Substituenten aus der Gruppe α ausgewählt sein können;
m 0, 1, 2, 3 oder 4 ist und n 0, 1, 2 oder 3 ist;
B und B' unabhängig voneinander aus einer der folgenden Gruppen a), b) oder c) ausgewählt sind, wobei
a) mono-, di- und trisubstituierte Arylreste sind, wobei der Arylrest Phenyl, Naphthyl oder Phenanthryl ist;
b) unsubstituierte, mono- und disubstituierte Heteroarylreste sind, wobei der Heteroarylrest Pyridyl, Furanyl, Benzofuranyl, Thienyl, Benzothienyl, 1,2,3,4-Tetrahydrocarbazolyl oder Julolidinyl ist;
wobei die Substituenten der Aryl- oder Heteroarylreste in a) und b) solche sind, ausgewählt aus der vorstehend definierten Gruppe α oder der Gruppe χ, bestehend aus Hydroxy, am Phenylring un-, mono- oder disubstituiertem 2-Phenylethenyl, am Phenylring un-, mono- oder disubstituiertem (Phenylimino)methylen, am Phenylring un-, mono- oder disubstituiertem (Phenylmethylen)imino, Amino, Mono-(C₁-C₆)-alkylamino, Di-(C₁-C₆)-alkylamino, am Phenylring un-, mono- oder disubstituiertem Mono- und Diphenylamino, Piperidinyl, N-substituiertem Piperazinyl, Pyrrolidinyl, Imidazolidinyl, Pyrazolidinyl, Indolinyl, Morpholinyl, 2,6-Dimethylmorpholinyl, Thiomorpholinyl, Azacycloheptyl, Azacyclooctyl, un-, mono- oder disubstituiertem Phenothiazinyl, un-, mono- oder disubstituiertem Phenoxazinyl, un-, mono- oder disubstituiertem 1,2,3,4-Tetrahydrochinolinyl, un-, mono- oder disubstituiertem 2,3-Dihydro-1,4-benzoxazinyl, un-, mono- oder disubstituiertem 1,2,3,4-Tetrahydroisochinolinyl, un-, mono- oder disubstituiertem Phenazinyl, un-, mono- oder disubstituiertem Carbazolyl, un-, mono- oder disubstituiertem 1,2,3,4-Tetrahydrocarbazolyl und un-, mono- oder disubstituiertem 10,11-Dihydrodibenz[b,f]azepinyl, wobei der oder die Substituenten unabhängig voneinander wiederum aus (C₁-C₆)-Alkyl, (C₁-C₆)-Alkoxy, Brom, Chlor oder Fluor ausgewählt sein können;
oder wobei zwei direkt benachbarte Substituenten eine Y-(CX₂)ₚ-Z-Gruppierung darstellen, wobei p = 1, 2 oder 3 ist, X Wasserstoff, CH₃ oder C₆H₅ sein kann und Y und Z unabhängig voneinander Sauerstoff, Schwefel, N-(C₁- C₆)-Alkyl, N-C₆H₅, CH₂, C(CH₃)₂ oder C(C₆H₅)₂ sein können, wobei zwei oder mehrere benachbarte Kohlenstoffatome dieser Y-(CX₂)ₚ-Z-Gruppierung jeweils unabhängig voneinander auch Teil eines daran annellierten Benzo-Ringsystems sein können, welches jeweils wiederum einen oder mehrere Substituenten, ausgewählt aus der Gruppe α oder der Gruppe χ, aufweisen kann;
oder
c) B und B' zusammen mit dem benachbarten Kohlenstoffatom des Pyran-Ringes einen un-, mono- oder disubstituierten 9,10-Dihydroanthracen-, Fluoren-, Thioxanthen-, Xanthen-, Benzo[b]fluoren-, 5H-Dibenzo[a,d]cyclohepten oder Dibenzosuberon-Rest oder einen gesättigten Kohlenwasserstoffrest, der (C₃-C₁₂)-spiro-monozyklisch, (C₇-C₁₂)-spiro-bizyklisch bzw. (C₇- C₁₂)-spiro-trizyklisch ist, bilden, wobei die Substituenten der ungesättigten Cyclen unabhängig voneinander aus der Gruppe α oder der Gruppe χ ausgewählt sein können.

2. Photochrome Naphthopyrane mit doppelt verbrückter Terphenyl-Untereinheit nach Anspruch 1, wobei die Reste R₅, R₆, R₇ und R₈ jeweils unabhängig voneinander aus der Gruppe α ausgewählt.

3. Photochrome Naphthopyrane mit doppelt verbrückter Terphenyl-Untereinheit nach Anspruch 1 oder 2, wobei B und B' unabhängig voneinander aus der Gruppe a) ausgewählt sind.

4. Photochrome Naphthopyrane mit doppelt verbrückter Terphenyl-Untereinheit nach einem der Änsprüche 1 bis 3, wobei die Reste R₅, R₆, R₇ und R₈ jeweils unabhängig voneinander aus der Gruppe α ausgewählt sind.

5. Photochrome Naphthopyrane mit doppelt verbrückter Terphenyl-Untereinheit nach einem der Ansprüche 1 bis 4, wobei die Reste R₁₃, R₁₄, R₁₅ und R₁₆ jeweils unabhängig voneinander aus der Gruppe α ausgewählt sind.

6. Photochrome Naphthopyrane mit doppelt verbrückter Terphenyl-Untereinheit nach einem der Ansprüche 1 bis 5, welche die nachfolgenden allgemeinen Formeln (II) bzw. (III) aufweisen: 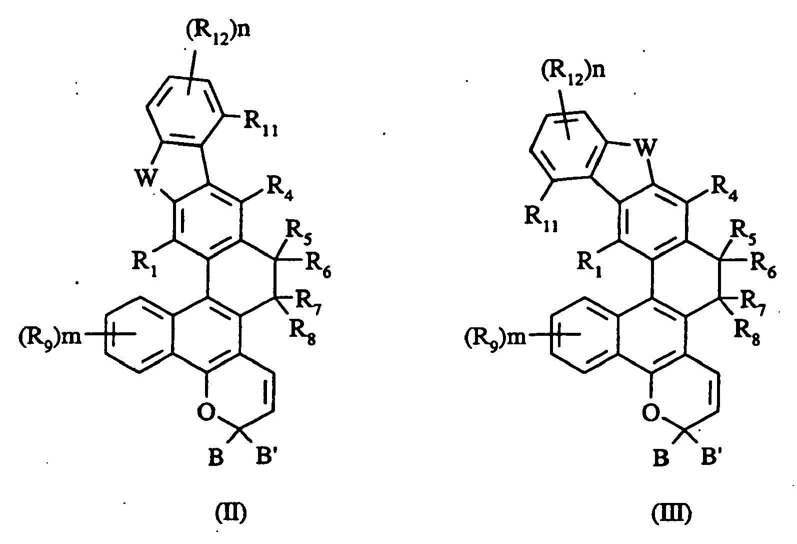 worin m, n, B, B', R₁ sowie R₄ bis R₁₂ wie vorgenannt definiert sind und W die vorstehend angeführte Verbrückung symbolisiert.

7. Verwendung der photochromen Naphthopyrane mit doppelt verbrückter Terphenyl-Untereinheit nach einem der Ansprüche 1 bis 6 in und auf Kunststoffmaterialien.

8. Verwendung nach Anspruch 7, wobei das Kunststoffmaterial eine ophthalmische Linse ist.

## Claims

1. Photochromic naphthopyranes with double-bridged terphenyl sub-unit with the general formula (I): 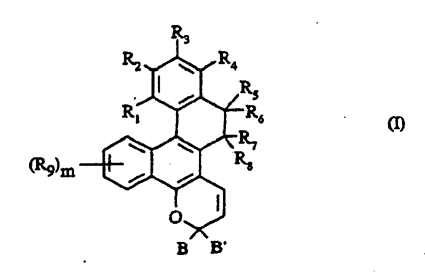 wherein
at least one of the residues R₂, R₃ or R₄, preferably R₂ or R₃, represents the following unit (A) 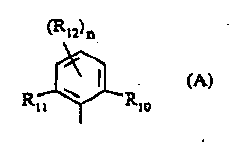 on condition that R₁₀ or R₁₁ together with a residue R₁, R₂, R₃ or R₄ that is ortho to the coupling point forms a bridge, or in the case of a coupling of the aforementioned unit (A) by means of R₂, R₁₀ and R₁₁ respectively together with both residues R₁ and R₃ ortho to the coupling point, or in the case of a coupling of the aforementioned unit (A) by means of R₃ with both residues R₂ and R₄ ortho to the coupling point form two bridges, wherein the bridging by means of residues R₁₀ or R₁₁ is respectively such a one selected from the group consisting of -CR₁₃R₁₄-, -O-, -S-, -N(Ph)-, -N(C₁-C₆ alkyl)-, -O-CR₁₃R₁₄-, S-CR₁₃R₁₄-, -CR₁₃R₁₄-CR₁₃R₁₄-, -CR₁₅=CR₁₆- or -CR₁₅=N-,
and wherein
residues R₁, R₂, R₃, R₄, R₁₀ and R₁₁, so long as they do not form a bridge, and also residues R₉ and R₁₂ respectively independently of one another represent a substituent selected from group α consisting of a hydrogen atom, a (C₁-C₆) alkyl residue, a (C₁-C₆) thioalkyl residue, a (C₃-C₇) cycloalkyl residue that can have one or more heteroatoms such as O or S, for example, a (C₁-C₆) alkoxy residue, a hydroxy group, a trifluoromethyl group, bromine, chlorine, fluorine, an un-, mono- or disubstituted phenyl-, phenoxy-, benzyl-, benzyloxy-, naphthyl- or naphthoxy residue, wherein the substituents can in turn be selected from group α; or
in the case of a coupling of the aforementioned unit (A) by means of R₄ residues R₁ and R₂ represent an annelated, unsubstituted, mono- or disubstituted benzo-, naphtho- or pyrido-ring, the substituents of which can be selected from group α;
or two residues R₉ ortho to one another or two residues R₁₂ ortho to one another form a -D-(CH₂)ₖ-E- group or -D-(C(CH₃)₂)ₖ-E- group bonded to the aromatic ring where k = 1 or 2, wherein D and E are selected independently of one another from oxygen, sulphur, CH₂, C(CH₃)₂ or C(C₆(H₅)₂, and wherein a benzo-ring can in turn be annelated to this -D-(CH₂)ₖ-E- group;
or two residues R₉ ortho to one another or two residues R₁₂ ortho to one another represent an unsubstituted, mono- or disubstituted benzo- or pyrido-ring, the substituents of which can be selected from group α;
residues R₅, R₆, R₇ and R₈ are respectively selected independently of one another from group α, or
residues R₅ and R₆ or R₇ and R₈ together form with inclusion of the spiro-carbon atom a 3- to 8-member carbo- or heteromonocyclic spiro-ring, which possibly bears one or more substituents from group α, and to which one to three aromatic or heteroaromatic ring systems can be annelated, wherein the ring system is selected independently of one another from group β consisting of benzene, naphthaline, phenanthrene, pyridine, quinoline, furan, thiophene, pyrrole, benzofuran, benzothiophene, indole and carbazole, which can in turn be substituted with one or more substituents from group α, wherein two adjacent annelated ring systems can also be linked to one another by an ortho,ortho' bridge, or
residues R₅ and R₆, or R₇ and R₈ together form with the inclusion of the spiro-carbon atom a 7- to 12-member carbo-bicyclic spiro-ring or a 7- to 12-member carbo-tricyclic spiro-ring, which respectively can possibly bear one or more substituents from group α,
or
residues R₅ and R₆ together with R₄ represent an annelated, unsubstituted, mono- or disubstituted benzo-, naphtho- or pyrido-ring, the substituents of which can be selected from group α;
residues R₁₃, R₁₄, R₁₅ and R₁₆ respectively independently of one another represent a substituent selected from group α; or
residues R₁₃ and R₁₄ together with a residue R₁₂ meta to the coupling point represent an annelated, unsubstituted, mono- or disubstituted benzo-, naphtho- or pyrido-ring, the substituents of which can be selected from group α, wherein this is only possible in the case of a bridging by means of groups -O-CR₁₃R₁₄- or -S-CR₁₃R₁₄- when CR₁₃R₁₄ is bonded directly to the phenyl ring that bears R₁₂, or
in the case of a coupling of the above unit (A) by means of R₃ and a bridging by means of R₂, residues R₁₃ and R₁₄ together with R₁, or in the case of a coupling of the above unit (A) by means of R₄ and a bridging by means of R₃, residues R₁₃ and R₁₄ together with R₂, or in the case of a coupling of the above unit (A) by means of R₂ and a bridging by means of R₃, residues R₁₃ and R₁₄ together with R₄ represent an annelated, unsubstituted, mono- or disubstituted benzo-, naphtho- or pyrido-ring, the substituents of which can be selected from group α, or
in the case of a coupling of the above unit (A) by means of R₃ and a bridging by means of R₄, residues R₁₃ and R₁₄ together with R₅ and R₆ represent an annelated, unsubstituted, mono- or disubstituted benzo-, naphtho- or pyrido-ring, the substituents of which can be selected from group α, wherein this is only possible in the case of a bridging by means of groups -O-CR₁₃R₁₄- or -S-CR₁₃R₁₄- when CR₁₃R₁₄ is bonded directly to the phenyl ring that also bears CR₅R₆, or
residues R₁₅ and R₁₆ represent an annelated, unsubstituted, mono- or disubstituted benzo-, naphtho- or pyrido-ring, the substituents of which can be selected from group α;
m is 0, 1, 2, 3 or 4 and n is 0, 1, 2 or 3;
B and B', independently of one another are selected from the following groups a), b) or c), wherein
a) are mono-, di- and trisubstituted aryl residues, wherein the aryl residue is phenyl, naphthyl or phenanthryl;
b) are unsubstituted, mono- and disubstituted heteroaryl residues, wherein the heteroaryl residue is pyridyl, furanyl, benzofuranyl, thienyl, benzothienyl, 1,2, 3,4-tetrahydrocarbazolyl or julolidinyl;
wherein the substituents of the aryl or heteroaryl residues in a) and b) are those selected from the above-defined group α or group χ consisting of hydroxy, 2-phenylethenyl un-, mono- or disubstituted on the phenyl ring, (phenylimino)methylene un-, mono- or disubstituted on the phenyl ring, (phenylmethylene) imino un-, mono- or disubstituted on the phenyl ring, amino, mono-(C₁-C₆) alkyl amino, di-(C₁-C₆) alkyl amino, mono- and disubstituted and diphenylamino un-, mono- or disubstituted on the phenyl ring, piperidinyl, N-substituted piperazinyl, pyrrolidinyl, imidazolidinyl, pyrazolidinyl, indolinyl, morpholinyl, 2,6-dimethylmorpholinyl, thiomorpholinyl, azacycloheptyl, azacyclooctyl, un-, mono- or disubstituted phenothiazinyl, un-, mono- or disubstituted phenoxazinyl, un-, mono- or disubstituted 1,2, 3,4-tetrahydroquinolinyl, un-, mono- or disubstituted 2,3-dihydro-1,4-benzoxazinyl, un-, mono- or disubstituted 1,2,3,4-tetrahydroisoquinolinyl, un-, mono- or disubstituted phenazinyl, un-, mono- or disubstituted carbazolyl, un-, mono- or disubstituted1,2,3,4-tetrahydrocarbazolyl and un-, mono- or disubstituted10,11-dihydrodibenz[b,f]azepinyl, wherein the substituent or substituents, independently or one another, in turn can be selected from (C₁-C₆) alkyl, (C₁-C₆) alkoxy, bromine, chlorine or fluorine;
or wherein two directly adjacent substituents represent a Y-(CX₂)ₚ-Z grouping, wherein p = 1, 2 or 3, X can be hydrogen, CH₃ or C₆H₅ and Y and Z independently of one another can be oxygen, sulphur, N-(C₁-C₆) alkyl, N-C₆H₅, CH₂, C(CH₃)₂ or C(C₆H₅)₂, wherein two or more adjacent carbon atoms of this Y-(CX₂)ₚ-Z grouping, respectively independently of one another, can also be a part of a benzo-ring system annelated thereto, which in turn can respectively have one or more substituents selected from group α or group χ; or
c) B and B' together with the adjacent carbon atom of the pyran ring form an un-, mono or disubstituted 9,10-dihydroanthracene, fluorene, thioxanthene, xanthene, benzo[b]fluorene, 5H-dibenzo[a,d]cycloheptene or dibenzosuberone residue or a saturated hydrocarbon residue, which is (C₃-C₁₂) spiro-monocyclic, (C₇-C₁₂) spiro-bicyclic or (C₇-C₁₂) spiro-tricyclic, wherein the substituents of the unsaturated cycles can be selected independently of one another from group α or group χ.

2. Photochromic naphthopyranes with double-bridged terphenyl sub-unit according to claim 1, wherein residues R₅, R₆, R₇ and R₈ are respectively selected independently of one another from group α.

3. Photochromic naphthopyranes with double-bridged terphenyl sub-unit according to claim 1 or 2, wherein B and B' are respectively selected independently of one another from group a).

4. Photochromic naphthopyranes with double-bridged terphenyl sub-unit according to one of claims 1 to 3, wherein residues R₅, R₆, R₇ and R₈ are respectively selected independently of one another from group α.

5. Photochromic naphthopyranes with double-bridged terphenyl sub-unit according to one of claims 1 to 4, wherein residues R₁₃, R₁₄, R₁₅ and R₁₆ are respectively selected independently of one another from group α.

6. Photochromic naphthopyranes with double-bridged terphenyl sub-unit according to one of claims 1 to 5, which have the following general formulae (II) or (III): 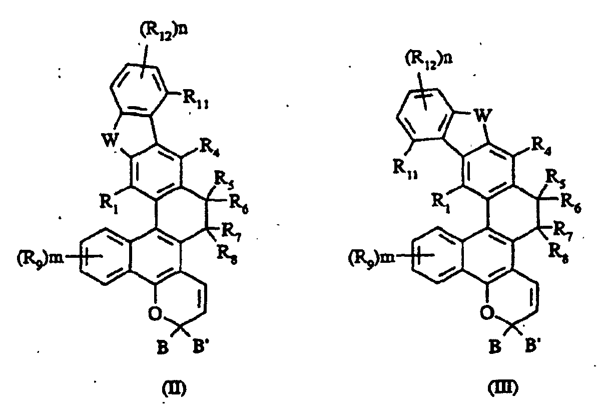 wherein m, n, B, B', R₁ and also R₄ to R₁₂ are defined as indicated above and W symbolises the bridging indicated above.

7. Use of the photochromic naphthopyranes with double-bridged terphenyl sub-unit according to one of claims 1 to 6 in and on plastic materials.

8. Use according to claim 7, wherein the plastic material is an ophthalmic lens.

## Revendications

1. Naphtopyranes photochromes comprenant une sous-unité terphényle doublement pontée de la formule générale (I) : 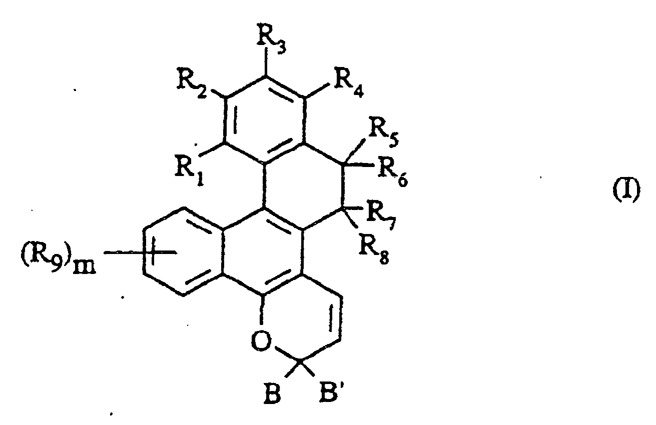 dans laquelle
au moins l'un des groupements R₂, R₃ ou R₄, de préférence R₂ ou R₃, représente l'unité (A) suivante 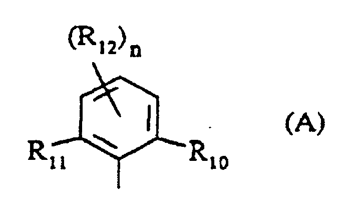 dans la mesure où R₁₀ ou R₁₁ forment ensemble un pontage avec un groupement R₁, R₂, R₃ ou R₄ en position ortho en tant que site de couplage, ou R₁₀ et R₁₁ forment deux pontages, dans le cas d'un couplage de l'unité précédente (A) par l'intermédiaire du groupement R₂, éventuellement, forment ensemble avec deux groupements R₁ et R₃ en position ortho en tant que sites de couplage, dans le cas d'un couplage de l'unité précédente (A) par l'intermédiaire de R₃ avec deux groupements R₂ et R₄ en position ortho en tant que sites de couplage, le pontage par les groupements R₁₀, respectivement R₁₁, étant un pontage semblable choisi dans le groupe constitué par -CR₁₃R₁₄-, -O-, -S-, -N(Ph)-,-N(alkyle en C₁-C₆)-, -O-CR₁₃R₁₄-, -S-CR₁₃R₁₄-, - CR₁₃R₁₄-CR₁₃R₁₄-, -CR₁₅=CR₁₆-, ou -CR₁₅=N-,
et dans laquelle
les groupements R₁, R₂, R₃, R₄, R₁₀ et R₁₁, tant qu'ils ne forment pas un pontage, ainsi que les groupements R9 et R12, éventuellement indépendamment les uns des autres, représentent un substituant choisi dans le groupe α, constitué d'un atome d'hydrogène, d'un groupement alkyle en (C₁-C₆), d'un groupement thioalkyle en (C₁-C₆), d'un groupement cycloalkyle en (C₃-C₇), qui peut présenter un ou plusieurs hétéroatomes, comme, par exemple, 0 ou S, un groupement alcoxy en (C₁-C₆), un groupement hydroxyle, un groupement trifluorométhyle, un atome de brome, de chlore, de fluor, un groupement phényle, phénoxy, benzyle, benzyloxy, naphtyle ou naphtoxy non, mono- ou di- substitué, les substituants pouvant à leur tour être choisis dans le groupe α ; ou
les groupements R₁ et R₂, dans le cas d'un couplage de l'unité précédente (A) par l'intermédiaire de R₄, représentent un groupement benzo, naphto ou pyrido cyclique condensé, non substitué, mono- ou di-substitué, dont les substituants peuvent être choisis dans le groupe α ;
ou deux groupements R₉ en position ortho l'un par rapport à l'autre, respectivement deux groupements R₁₂ en position l'un par rapport à l'autre, forment un groupement -D-(CH₂)ₖ-E-, ou -D-{C(CH₃)₂]}ₖ-E-, liés au cycle aromatique, avec k = 1 ou 2, où D et E sont choisis indépendamment l'un de l'autre parmi de l'oxygène, du soufre, des groupements CH₂, C(CH₃)₂ ou C(C₆H₅)₂, et où un cycle benzénique peut être à nouveau condensé sur ce groupement -D-(CH₂)ₖ-E ;
ou deux groupements R₉ en position ortho l'un par rapport à l'autre , respectivement deux groupements R₁₂ en position ortho l'un par rapport à l'autre, représentent un cycle benzo ou pyrido non, mono- ou di-substitué dont les substituants peuvent être choisis dans le groupe α ;
les groupements R₅, R₆, R₇ et R₈ sont choisis indépendamment les uns des autres dans le groupe α, ou
les groupements R₅ et R₆, respectivement R₇ et R₈, forment ensemble un cycle spiro hétéromonocyclique ou avec de 3 à 8 chaînons carbonés, moyennant l'intégration de l'atome de carbone du groupement spiro, qui porte éventuellement un ou plusieurs substituants du groupe α, sur lequel de un à trois systèmes cycliques aromatiques ou hétéroaromatiques peuvent être condensés, le système cyclique pouvant être choisi, indépendamment l'un de l'autre, dans le groupe ß constitué du benzène, du naphtalène, du phénanthrène, de la pyridine, de la quinoléine, du furane, du thiophène, du pyrolle, du benzofurane, du benzothiophène, de l'indole et du carbazole, qui à leur tour, peuvent être substitués par un ou plusieurs substituants du groupe α, deux systèmes cycliques voisins condensés pouvant également être liés ensemble par un pont ortho, ortho', ou
les groupements R₅ et R₆, respectivement R₇ et R₈, forment ensemble un cycle spiro bicyclique de 7 à 12 chainons carbonés, ou un cycle spiro tricyclique de 7 à 12 chaînons carbonés, moyennant l'intégration de l'atome de carbone du groupement spiro, qui peuvent chacun éventuellement porter un ou plusieurs substituants du groupe α,
ou
les groupements R₅ et R₆ représentent, ensemble avec R₄, un cycle benzo, naphto ou pyrido condensé, non substitué, mono- ou di-substitué, dont les substituants peuvent être choisis dans le groupe α,
les groupements R₁₂, R₁₄, R₁₅ et R₁₆ représentent, éventuellement indépendamment les uns des autres, un substituant choisi dans le groupe α, ou
les groupements R₁₃ et R₁₄ représentent, ensemble avec un groupement R₁₂ en position méta en tant que site de couplage, un cycle benzo, naphto ou pyrido condensé, non substitué, mono- ou di-substitué, dont les substituants peuvent être choisis dans le groupe α, ceci n'étant possible qu'en cas de pontage au moyen des groupements -O-CR₁₃R₁₄-, respectivement -S-CR₁₃R₁₄-, lorsque CR₁₃R₁₄ est relié directement au cycle phényle qui porte le groupement R₁₂, ou
dans le cas d'un couplage de l'unité précédente (A) par l'intermédiaire de R₃ et d'un pontage par l'intermédiaire de R₂, les groupements R₁₃ et R₁₄ représentent, ensemble avec R₁, respectivement dans le cas d'un couplage de l'unité précédente (A) par l'intermédiaire de R₄ et d'un pontage par l'intermédiaire de R₃, les groupements R₁₃ et R₁₄ représentent, ensemble avec R₂, respectivement dans le cas d'un couplage de l'unité (A) précédente par l'intermédiaire de R₂ et d'un pontage par l'intermédiaire de R₃, les groupements R₁₃ et R₁₄, ensemble avec R₄, représentent un cycle benzo, naphto ou pyrido condensé, non substitué, mono- ou di-substitué, dont les substituants peuvent être choisis dans le groupe α, ou
dans le cas d'un couplage de l'unité (A) précédente par l'intermédiaire de R₃ et d'un pontage par l'intermédiaire de R₄, les groupements R₁₃ et R₁₄, représentent, ensemble avec R₅ et R₆, un cycle benzo, naphto, ou pyrido condensé, non substitué, mono- ou di-substitué, dont les substituants peuvent être choisis dans le groupe α, ceci étant possible uniquement pour un pontage au moyen des groupements -O-CR₁₃R₁₄-, respectivement -S-CR₁₃R₁₄-, lorsque le groupement CR₁₃R₁₄ est directement relié au cycle phényle qui porte également le groupement CR₅R₆, ou
les groupements R₁₅ et R₁₆ représentent un cycle benzo, naphto ou pyrido condensé, non substitué, mono- ou di-substitué, dont les substituants peuvent être choisis dans le groupe α ;
m est égal à 0, 1, 2, 3 ou 4 et n est égal à 0, 1, 2 ou 3 ;
B et B' sont choisis indépendamment l'un de l'autre dans l'un des groupes suivants a), b) ou c), où
a) représente des groupements aryles mono-, di- ou tri-substitués, le groupement aryle étant un groupement phényle, un groupement naphtyle ou un groupement phénanthryle ;
b) représente des groupements hétéroaryles non substitués, mono- et di-substitués, le groupement hétéroaryle étant le groupement pyridyle, le groupement furanyle, le groupement benzofuranyle, le groupement thiényle, le groupement benzothiényle, le groupement 1,2,2,4-tétrahydrocarbazolyle ou le groupement julolidinyle ;
les substituants des groupements aryles ou hétéroaryles dans les groupes a) et b) étant ceux choisis dans le groupe α, défini précédemment, ou dans le groupe χ, constitué du groupement hydroxyle, sur un cycle phényle non, mono- ou di-substitué 2-phényléthényle, sur le cycle phényle non, mono ou di-substitué (phénylimino)méthylène, sur un cycle phényle non, mono- ou di-substitué d'un groupement (phénylméthylèn)imino, amine, mono alkylamine en (C₁-C₆), di-alkylamine en (C₁-C₆), sur un cycle phényle non, mono- ou di-substitué diphénylamine, pipéridinyle, pipérazinyle substitué en N, pyrrolidinyle, imidazolidinyle, pyrazolidinyle, indolinyle, morpholinyle, 2,6-diméthylmorpholinyle, thiomorpholinyle, azacycloheptyle, azacyclooctyle, phénothiazinyle non, mono- ou di-substitué, phénoxazinyle non, mono- ou di-substitué, 1,2,3,4-tétrahydroquinolinyle non, mono- ou di-substitué, 2,3-dihydro-1,4-benzoxazinyle non, mono- ou di-substitué, 1,2,3,4-tétrahydroisoquinolinyle non, mono- ou di-substitué, phénazinyle non, mono- ou di-substitué, carbazolyle non, mono- ou di-substitué, 1,2,3,4-tétrahydrocarbazolyle non, mono- ou di-substitué et 10,11-dihydrodibenz[b,f]azépinyle non, mono- ou di-substitué, le ou les substituants pouvant, à leur tour, être choisis, indépendamment les uns des autres, dans le groupe constitué par un groupement alkyle en (C₁-C₆), un groupement alcoxy en (C₁-C₆), un atome de brome, un atome de chlore ou un atome de fluor ;
ou deux substituants directement voisins représentant un groupement Y-(CX₂)ₚ-Z-, où p = 1, 2 ou 3, X pouvant être un atome d'hydrogène, un groupement CH₃ ou un groupement C₆H₅, et Y et Z pouvant être, indépendamment l'un de l'autre, de l'oxygène, du soufre, un groupement N-alkyle en (C₁-C₆) , un groupement N-C₆H₅, un groupement CH₂, un groupement C(CH₃)₂ ou un groupement C(C₆H₅)₂, deux ou plusieurs atomes de carbone voisins de ces groupements Y-(CX₂)ₚ-Z pouvant être, éventuellement indépendamment les uns des autres, également une partie d'un système de cycle benzo qui y est condensé, qui encore, peut présenter un ou plusieurs substituants choisis dans le groupe α ou dans le groupe χ ;
ou
c) B et B' forment, ensemble avec l'atome de carbone du cycle pyrane voisin, un groupement 9,10-dihydroanthracène non, mono- ou di-substitué, un groupement fluorène, un groupement thioxanthène, un groupement xanthène, un groupement benzo[b]fluorène, un groupement 5H-dibenzo[a,d]cycloheptène ou un groupement dibenzosubérone ou un groupement hydrocarboné saturé qui est spiro monocyclique en (C₃-C₁₂), spiro bicyclique en (C₇-C₁₂), respectivement spiro tricyclique en (C₇-C₁₂), les substituants des cycles non saturés pouvant être choisis, indépendamment les uns des autres, dans le groupe α ou dans le groupe χ.

2. Naphtopyranes photochromes comprenant une unité terphényle doublement pontée selon la revendication 1, les groupements R₅, R₆, R₇ et R₈ étant choisis, indépendamment les uns des autres, dans le groupe α.

3. Naphtopyranes photochromes comprenant une unité terphényle doublement pontée selon les revendication 1 ou 2, B et B' étant choisis indépendamment l'un de l'autre dans le groupe a).

4. Naphtopyranes photochromes comprenant une unité terphényle doublement pontée selon l'une des revendications 1 à 3, les groupements R₅, R₆, R₇ et R₈ étant, éventuellement indépendamment les des autres, choisis dans le groupe α.

5. Naphtopyranes photochromes comprenant une unité terphényle doublement pontée selon l'une des revendications 1 à 4, les groupements R₁₃, R₁₄, R₁₅ et R₁₆ étant, éventuellement indépendamment les uns des autres, choisis dans le groupe α.

6. Naphtopyranes photochromes comprenant une unité terphényle doublement pontée selon l'une des revendications 1 à 5 qui présente les formules générales (II), respectivement (III) : 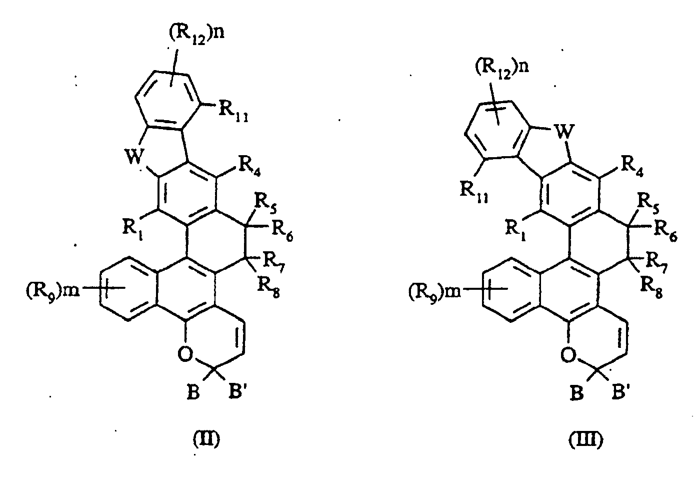 dans lesquelles m, n, B, B', R₁, ainsi que les groupements R₄ à R₁₂, sont définis comme précité et W symbolise le pontage énoncé précédemment.

7. Utilisation des naphtopyranes photochromes comprenant une unité terphényle doublement pontée selon l'une des revendications 1 à 6 dans, et sur, des matériaux en matière synthétique.

8. Utilisation selon la revendication 7, le matériau en matière synthétique étant une lentille ophtalmique.
